# EUROPEAN PATENT SPECIFICATION

(11) **EP 2 523 935 B1**
(45) Date of publication and mention of the grant of the patent: **02.12.2015**
(21) Application number: 11705301.7
(22) Date of filing: 11.01.2011
(51) Int. Cl.: C07C 309/24, C10M 135/10

(54) **OVERBASED ALKYLATED ARYLALKYL SULFONATES**
ÜBERBASISCHE ALKYLIERTE ARYLALKYL-SULFONATE
ARYLALKYL SULFONATES ALKYLÉS SURALCALINISÉS

(30) Priority: 11.01.2010 US 293732 P
(43) Date of publication of application: 21.11.2012
(73) Proprietor: The Lubrizol Corporation, Wickliffe OH 44092-2298 (US)
(72) Inventor: ROSKI, James P., Wickliffe Ohio 44092-2298 (US); DELBRIDGE, Ewan E., Wickliffe Ohio 44092-2298 (US); FRIEND, Christopher, Belper Derbyshire DE56 1QN (GB); WALKER, Gary M., Belper Derbyshire DE56 1QN (GB); CARRICK, Virginia A., Wickliffe Ohio 44092-2298 (US); VILARDO, Jonathan, Wickliffe, Ohio 44092-2298 (US)
(74) Representative: D Young & Co LLP
(86) International application number: PCT/US2011/020736
(87) International publication number: WO 2011/085339

(56) References cited:
- US-A- 3 422 161
- US-A1- 2009 023 950
- US-A1- 2009 023 951

## Description

### BACKGROUND OF THE INVENTION

The disclosed technology relates to overbased alkylated arylalkyl sulfonates that are useful as detergents in lubricant applications.

U.S. Patent Publication 2009/0023950, Berger et al., January 22, 2009, discloses polyalkylated arylalkyl sulfonic acids and their salts. Such materials are reported to have the general structure 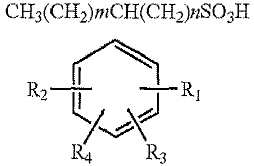 where R₁, R₂ and R₃ are each separately and independently H, alkyl (branched or linear C1 to C30), R₄= CH₃(CH₂)ₒCH(CH₂)ₚCH₃, m+n =4 to 28, and o+p = 3 to 27. The material is optionally neutralized with a base such as sodium hydroxide, sodium carbonate, potassium hydroxide, potassium carbonate, calcium hydroxide, magnesium hydroxide, ammonium hydroxide, or amines.

U.S. Patent Publication 2009/0023951, Berger et al., January 22, 2009, discloses a process for producing polyalkylated arylalkyl sulfonic acids. The structure of the polyalkylated arylalkyl sulfonic acids produced using the process described therein is 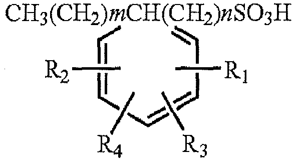 where R₁, R₂ and R₃ are each separately and independently H, alkyl (branched or linear C1 to C30), (-CH₂CH₂O)*ₐ*, (-CH(CH₃)CH₂O)*_{b}*, or (-CH₂CH₂O)c(-CH₂(CH₃)CH₂O)*_{d}*, m+n=8 to 28, R₄=CH₂CH₃, CH₂CH₃Y, or Y(CH₂)ₚCH(CH₂)*_{q}*Y, p+q = 0 to 27, a=1 to 30, b=1 to 30, c+d=2 to 30, and Y=CH₃, COOH, CH₂OH, CH₂(-CH₂CH₂O)*ₐ*, CH₂(-CH(CH₃)CH₂O)*_{b}*, CH₂(-CH₂CH₂O)c(-CH₂(CH₃)CH₂O)*_{d}*, aromatic, or substituted aromatic. The poly alkylated arylalkyl sulfonic acids may be further neutralized with alkalis or amines to form the corresponding sulfonated salts.

U.S. Patent 3,488,284, LeSuer et al., January 6, 1970, discloses basic metal complexes obtained by treating an oil-soluble acid such as sulfonic acid with a metal base in the presence of an acidic gas and an alcoholic promoter. The complexes are said to be useful as detergent additives in fuels, oils and other organic composition, and especially useful in lubricants. The oil-soluble organic compound may be either aromatic, aliphatic, cycloaliphatic, or arylaliphatic.

The disclosed technology, therefore, may solve one or more of the problems encountered in lubricants and detergents for lubricants, including providing one or more of good viscosity performance, reduced coefficient of friction, improved wear performance, improved soot-handling/dispersion performance, improved fuel economy, improved engine durability, lighter color, ease of preparation of an overbased sulfonate detergent, and increased molecular weight without encountering properties of waxiness. Good or improved performance may also be obtained in one or more of deposit formation, foaming behavior, demulsification behavior, and gelling properties.

### SUMMARY OF THE INVENTION

The disclosed technology provides an overbased salt comprising:
an anionic portion represented by 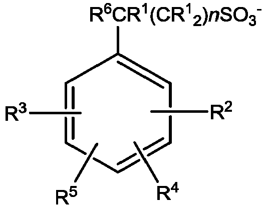 wherein each R¹ is independently H or an alkyl group of 1 to 4 carbon atoms; R², R³, and R⁴ are independently H or hydrocarbyl groups, R⁵ is a group represented by -CR¹R⁷(CR¹₂)*ₒ*CHR¹₂, R⁶ is H or -(CR¹₂)*ₘ*CHR¹₂, and R⁷ is H or -(CR¹₂)*ₚ*CHR¹₂ ; *m*, *n, o*, and *p* are numbers such that *m*+*n* is 4 to 28 and *o+p* is 3 to 27, provided that when R⁶ is H, *m* is defined as 0 and *n* is 5 to 29 and when R⁷ is H, *p* is defined as 0 and *o* is 4 to 28; and wherein the total number of carbon atoms in said anionic portion is at least 26; and a metal or amine or ammonium cation; wherein the overbased salt has a cation content in excess of that which would be present for neutralization according to the stoichiometry of the cation and of said anionic portion.

The technology also provides a method of preparing an overbased salt, comprising combining a sulfonic acid represented by 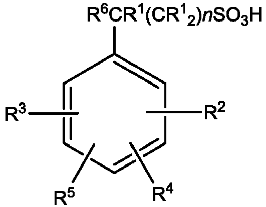 where *n*, *m*, *o*, *p*, R¹, R², R³, R⁴, R⁵, and R⁶ are as defined above with a stoichiometric excess of a basic metal compound or amine compound or ammonia and optionally reacting said mixture with carbon dioxide. The ammonia may be supplied in the form of, e.g., ammonium hydroxide.

Further provided is a lubricant composition comprising an oil of lubricating viscosity and the overbased salt described above, as well as a method for lubricating a mechanical device, comprising supplying thereto such a lubricant.

### DETAILED DESCRIPTION OF THE INVENTION

Various preferred features and embodiments will be described below.

The present technology relates to an overbased salt as described herein. Overbased materials, otherwise referred to as overbased or superbased salts, are generally homogeneous Newtonian systems characterized by a metal content in excess of that which would be present for neutralization according to the stoichiometry of the metal and the particular acidic organic compound reacted with the metal. The overbased materials in general are prepared by reacting an acidic material (typically an inorganic acid or lower carboxylic acid, preferably carbon dioxide) with a mixture comprising an acidic organic compound, a reaction medium comprising at least one inert, organic solvent (e.g., mineral oil, naphtha, toluene, xylene) for said acidic organic material, a stoichiometric excess of a metal base or alternatively of another basic material such as ammonia or an amine, and a promoter such as a phenol or alcohol. The acidic organic material will normally have a sufficient number of carbon atoms, for instance, as a hydrocarbyl substituent, to provide a reasonable degree of solubility in oil. The amount of excess metal is commonly expressed in terms of metal ratio. The term "metal ratio" is the ratio of the total equivalents of the metal (if the neutralizing material is a basic metal) to the equivalents of the acidic organic compound. A neutral metal salt has a metal ratio of one. A salt having 4.5 times as much metal as present in a normal salt will have metal excess of 3.5 equivalents, or a ratio of 4.5. The term "metal ratio" may also be loosely applied, by analogy, to materials which have an ammonium or amine cation, rather than a metal cation.

Overbased detergents are often characterized by Total Base Number (TBN). TBN is the amount of strong acid needed to neutralize all of the overbased material's basicity, expressed as potassium hydroxide equivalents (mg KOH per gram of sample). Since overbased detergents are commonly provided in a form which contains a certain amount of diluent oil, for example, 40-50% oil, the actual TBN value for such a detergent will depend on the amount of such diluent oil present, irrespective of the "inherent" basicity of the overbased material. For the purposes of the present invention, the TBN of an overbased detergent is to be recalculated to an oil-free basis. Detergents which are useful in the present invention typically have a TBN (oil-free basis) of 100 to 1100, and in one embodiment 100 to 800, and in another 150 to 750, and in another, 400 to 700. If multiple detergents are employed, the overall TBN of the detergent component (that is, an average of all the specific detergents together) will typically be in the above ranges.

The overall TBN of a lubricant composition, including oil, will derived from the TBN contribution of the individual components, such as the dispersant, the detergent, and other basic materials. The overall TBN of a lubricant will typically be at least 7 or at least 10, or sometimes even at least 20. Sulfated ash (ASTM D-874) is another parameter often used to characterize such compositions. Certain of the lubricant compositions of the present technology can have sulfated ash levels of 0.5 to 5% or 0.8 to 4% or to 2%, for instance, greater than 0.8%, greater than 1.0%, or even greater than 2%.

The metal compounds useful in making the basic metal salts are generally any Group 1 or Group 2 metal compounds (CAS version of the Periodic Table of the Elements). The Group 1 metals of the metal compound include Group 1a alkali metals such as sodium, potassium, and lithium, as well as Group 1b metals such as copper. The Group 1 metals can be sodium, potassium, lithium and copper, and in one embodiment sodium or potassium, and in another embodiment, sodium. The Group 2 metals of the metal base include the Group 2a alkaline earth metals such as magnesium, calcium, and barium, as well as the Group 2b metals such as zinc or cadmium. In one embodiment the Group 2 metals are magnesium, calcium, barium, or zinc, and in another embodiments magnesium or calcium. In certain embodiments the metal is calcium or sodium or a mixture of calcium and sodium. Generally the metal compounds are delivered as metal salts. The anionic portion of the salt can be hydroxide, oxide, carbonate, borate, or nitrate. Commonly used basic metal compounds include calcium oxide and calcium hydroxide.

Overbased materials in general are well known to those skilled in the art. Patents describing techniques for making basic salts of sulfonic acids, carboxylic acids, (hydrocarbyl-substituted) phenols, phosphonic acids, and mixtures of any two or more of these include U.S. Patents 2,501,731; 2,616,905; 2,616,911; 2,616,925; 2,777,874; 3,256,186; 3,384,585; 3,365,396; 3,320,162; 3,318,809; 3,488,284; and 3,629,109.

The overbased materials of the present invention, however, are based on a sulfonic acid.

The overbased materials of the present technology may be formed from the polyalkylated arylalkyl sulfonic acids or their salts described in US 2009/0023950.

The sulfonic acid used in the present technology (providing the corresponding anionic group) may generally be represented by the structure

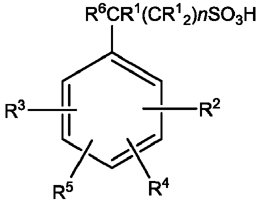

In this material, each of the R¹ groups may independently be hydrogen or an alkyl group of 1 to 4 carbon atoms, alternatively 1 to 2 carbon atoms. In certain embodiments some or all of the R¹ groups are methyl, such as when the upper substituent group and/or the R⁵ group are derived from propylene. In other embodiments some or all of the R¹ groups are hydrogen, such as when one or both of the aforementioned groups are derived from ethylene.

Groups R², R³, and R⁴ are each independently hydrogen or hydrocarbyl groups. The hydrocarbyl groups may each contain 1 to 30 carbon atoms, or 1 to 18 or 1 to 6 or 1 or 2 carbon atoms. The hydrocarbyl groups may be alkyl groups. They may be linear or branched, and they may be saturated or unsaturated (e.g., alkylene) groups. In one embodiment, one or two of groups R², R³, and R⁴ are methyl groups. In one embodiment R² and R³ are methyl groups and R⁴ is hydrogen. In one embodiment the alkylated product is prepared from a xylene.

R⁵ is a group represented by -CR¹R⁷(CR¹₂)*ₒ*CHR¹₂ wherein R⁷ is H or -(CR¹₂)*ₚ*CHR¹₂ and R¹ is defined as above. Within the R⁵ group, *o* and *p* are numbers such that *o*+*p* is 3 to 27 or 7 to 21 or 7 to 17 or 9 to 15 or 11 to 13, provided that when R⁷ is H, *p* is defined as 0 and each of the above ranges of *o* + *p* is increased by 1. (That is, when R⁷ is H, not only is there no internal linking group -CR¹₂-, but neither is there a carbon atom from the end group CHR¹₂, but the total number of carbon atoms may be unchanged) These ranges may also be appropriate when the R¹ groups are H; when they are alkyl groups the total of *o+p* may be reduced, if desired, by the number of carbon atoms provided by the R¹ groups within R⁵.

Thus, the total number of carbon atoms in the linear chain of carbon atoms in R⁵ will typically be 6 to 30 or 10 to 24 or 12 to 18 or 14 to 16. When all the R¹ groups within R⁵ are hydrogen, the R⁵ group is normally referred to as a "linear" group, even though the point of attachment of the group to the aromatic ring may not be at a terminal carbon. Such a R⁵ group may frequently be attached in the 2- position or the 3- (or higher) position, resulting in a methyl or ethyl (or higher) branch at the point of attachment, Attachment in the 1- position is also possible, but it is believed that 2- or 3- attachment may be more prominent. Thus a common representation of the attachment of a linear R⁵ group to an aromatic ring may be as shown: 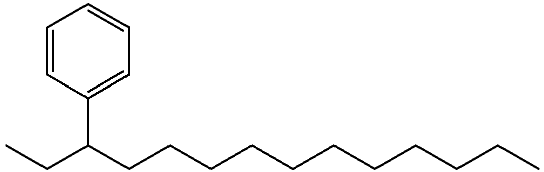 A branched R⁵ group, in contrast, would have branches along the chain, in addition to the nominal branch point at the position of attachment.

In the structure above, R⁶ is H or -(CR¹₂)*ₘ*CHR¹₂. In this regard, R⁶ is similar to R⁷, described above. In the upper, SO₃H containing, substituent 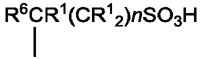 *m* and *n* are numbers such that *m*+*n* is 4 to 28 or 8 to 22 or 8 to 18 or 10 to 16 or 12 to 14, provided that when R⁶ is H, *m* is defined as 0 and the values for *m*+*n* are increased by 1 from those reported immediately above. (That is, when R⁶ is H, not only is there no internal linking group -CR¹₂-, but neither is there a carbon atom from the end group CHR¹₂, yet the total number of carbon atoms may remain the same) These ranges are also appropriate when the R¹ groups are H; when they are alkyl groups the total of *m*+*n* may be reduced, if desired, by the number of carbon atoms provided by the R¹ groups within the overall substituent. Thus, the total number of carbon atoms in the linear chain of carbon atoms in R⁶ will typically be 6 to 30 or 10 to 24 or 12 to 18 or 14 to 16. The discussion of "linear" in connection with the R⁵ group is also applicable here, given that the group may not be attached at the terminal carbon but may nevertheless be considered to be linear.

The total number of carbon atoms in the sulfonic acid or the resulting sulfonate, that is, in the anionic portion of the overbased salt, should be at least 26 or at least 30 or at least 34, and may be up to an upper value which is not clearly defined from a technical point of view but may, practically, be or 120 or 100 or 80 or 70 or 66. Suitable ranges include 30 to 60 or 34 to 50 or 36 to 40. The total number of carbon atoms includes the aromatic carbon atoms, which, for a benzene ring is 6, so the number of carbon atoms in the substituent groups would be, for instance, 6 less than the above numbers. It is believed that if the total number of carbon atoms is below 26, sulfonic acid may not exhibit sufficient oil-solubility to permit the overbasing process to proceed smoothly, particularly when the medium in which the process is conducted is oil.

In certain embodiments, the anionic portion of the overbased material may be represented by the structure 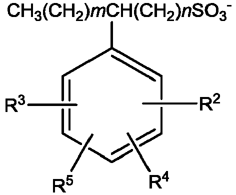 wherein R⁵ is represented by -CHR⁷CH₂)ₒCH₃ and R⁷ is H or -(CH₂)*ₚ*CH₃. More specific embodiments include species that may be represented by 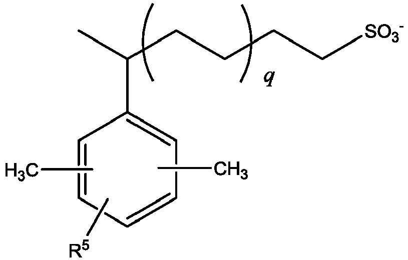 and isomers thereof, wherein q is 5 or 6 and R⁵ is a linear alkyl group containing 14 to 16 carbon atoms. Another exemplary representation could be 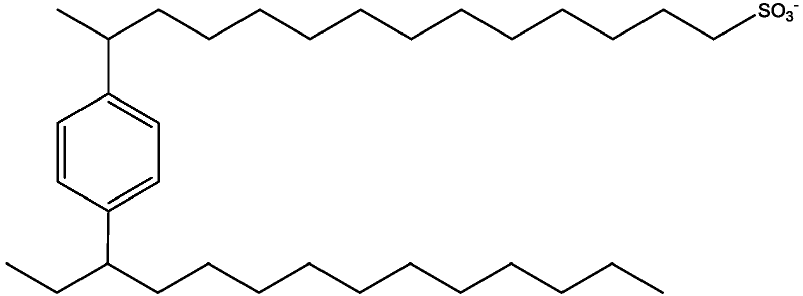 and positional isomers thereof (both in terms of location on the benzene ring and position of attachment onto the carbon chains). It is to be understood that a certain portion of the molecules may comprise di-sulfonated or more highly sulfonated materials. For instance, in some molecules (e.g., representing 0 to 10% or 0.5 to 5% or 1 to 4% of the mixture) each of the long-chain alkyl groups may bear an SO₃⁻ group. Similarly, there may be present a portion of dialkylated molecules without any SO₃⁻ group. Synthesis of the arylalkyl sulfonic acid is described in greater detail in U.S. Patent Publication 2009/0023950; see paragraphs 0025 through 0040.

The present overbased detergents may be prepared by methods generally known for preparing overbased detergents, as described above. More particularly, the appropriate sulfonic acid may be first converted to a neutral salt by reaction with a basic compound such as CaO, at room temperature or elevated temperature. The neutral salt may be subsequently overbased; or the neutralization and overbasing may occur in a single process.

The overbasing may include mixing of the neutral salt (or the precursor sulfonic acid) with a stoichiometric excess of basic compound (e.g., a basic metal compound such as calcium oxide or calcium hydroxide, or alternatively compounds such as, magnesium hydroxide, magnesium oxide, sodium hydroxide, or sodium oxide; or alternatively a basic nitrogen compound such as ammonia or an amine), typically in a solvent such as mineral oil, and typically in the presence of one or more promoters such as alcohols. Typical alcoholic promoters include mixtures of methanol, isobutyl alcohol, and/or amyl alcohols, in various proportions. Optionally a small amount of a dispersant (described below) or another detergent may be present. The mixture is treated with an acidic gas, such as typically CO₂, which will convert at least a portion of the excess basic compound to the salt, such as CaCO₃. The addition of the basic compound and the subsequent treatment with the acidic gas may be conducted in several portions or iterations, which may permit formation of materials with a higher metal ratio and total base number. After the reaction is complete, volatile components may be removed. The overbased materials may have metal ratios of 1.1 to 40, or 2 to 35, or 2.5 to 25, or 2.5 to 10, or 10 to 20 (or corresponding analogous values if a non-metal basic compound is used). The TBN of the products may be 10 to 1100 or 20 to 800 or 30 to 600 (calculated on an oil-free basis). The metal ion employed may typically include calcium, magnesium, or sodium (i.e., a calcium, magnesium, or sodium cation) or mixtures thereof, for example, calcium.

One of the advantages of the present products is revealed in their ease of preparation or stability characteristics. It is known that conventional alkaryl sulfonates may exhibit a problem of "skinning" during the overbasing process or upon storage thereafter. This problem is identified, for instance, in U.S. Patent 6,054,410. Here it is disclosed that a superficial "skin" may form, leading to high viscosity and low incorporation of calcium, among other difficulties. This problem was addressed by restricting the amount of aryl substitution on the 1 or 2 position of the linear hydrocarbyl chain, or by using a selected mixture of alkyl aryl sulfonates of super alkalinized (i.e., overbased) alkaline earth metals, that is, effectively diluting the mixture with heavy sulfonic acids. The materials of the present technology, however, do not appear to exhibit this problem at all. The materials of the present technology may also impart a measure of improvement, such as anti-skinning performance, during overbasing or storage, or other improvements. This may be observed when employed in combination with other substrates, e.g., as cosubstrates in an overbasing process, or when otherwise used in combination with other sulfonate substrates or phenates or other such materials as described hereinbelow in connection with the optional additional detergents.

Amount of the overbased material described herein, within a lubricant composition, will depend on the requirements of the particular lubricant. However, it may generally be 0.01 to 5 or to 8 percent by weight, or alternatively 0.1 to 4 or 0.3 to 3.5% or 0.5 to 3% or 0.8 to 2.5% by weight.

The present materials are typically used in combination with an oil of lubricating viscosity. The base oil used in the inventive lubricating oil composition may be selected from any of the base oils in Groups I-V as specified in the American Petroleum Institute (API) Base Oil Interchangeability Guidelines. The five base oil groups are as follows:

| Base Oil Category | Sulfur (%) | Saturates (%) | Viscosity Index |
|---|---|---|---|
| Group I | >0.03 and/or | <90 | 80 to 120 |
| Group II | ≤0.03 and | ≥90 | 80 to 120 |
| Group III | ≤0.03 and | ≥90 | >120 |
| Group IV | All polyalphaolefins (PAOs) | | |
| Group V | All others not included in Groups I, II, III or IV | | |

Groups I, II and III are mineral oil base stocks. The oil of lubricating viscosity, then, can include natural or synthetic lubricating oils and mixtures thereof. Mixture of mineral oil and synthetic oils, particularly polyalphaolefin oils and polyester oils, are often used.

Natural oils include animal oils and vegetable oils (e.g. castor oil, lard oil and other vegetable acid esters) as well as mineral lubricating oils such as liquid petroleum oils and solvent-treated or acid treated mineral lubricating oils of the paraffinic, naphthenic or mixed paraffinic-naphthenic types. Hydrotreated or hydrocracked oils are included within the scope of useful oils of lubricating viscosity.

Oils of lubricating viscosity derived from coal or shale are also useful. Synthetic lubricating oils include hydrocarbon oils and halosubstituted hydrocarbon oils such as polymerized and interpolymerized olefins and mixtures thereof, alkylbenzenes, polyphenyl, (e.g., biphenyls, terphenyls, and alkylated polyphenyls), alkylated diphenyl ethers and alkylated diphenyl sulfides and their derivatives, analogs and homologues thereof. Alkylene oxide polymers and interpolymers and derivatives thereof, and those where terminal hydroxyl groups have been modified by, for example, esterification or etherification, constitute other classes of known synthetic lubricating oils that can be used. Another suitable class of synthetic lubricating oils that can be used comprises the esters of dicarboxylic acids and those made from C5 to C12 monocarboxylic acids and polyols or polyol ethers.

Other synthetic lubricating oils include liquid esters of phosphorus-containing acids, polymeric tetrahydrofurans, silicon-based oils such as the polyalkyl-, polyaryl-, polyalkoxy-, or polyaryloxy-siloxane oils, and silicate oils.

Hydrotreated naphthenic oils are also known and can be used. Synthetic oils may be used, such as those produced by Fischer-Tropsch reactions and typically may be hydroisomerised Fischer-Tropsch hydrocarbons or waxes. In one embodiment oils may be prepared by a Fischer-Tropsch gas-to-liquid synthetic procedure as well as other gas-to-liquid oils.

Unrefined, refined and rerefined oils, either natural or synthetic (as well as mixtures of two or more of any of these) of the type disclosed hereinabove can used in the compositions of the present invention. Unrefined oils are those obtained directly from a natural or synthetic source without further purification treatment. Refined oils are similar to the unrefined oils except they have been further treated in one or more purification steps to improve one or more properties. Rerefined oils are obtained by processes similar to those used to obtain refined oils applied to refined oils which have been already used in service. Such rerefined oils often are additionally processed by techniques directed to removal of spent additives and oil breakdown products.

The amount of oil in a lubricant formulation may typically be the amount which, when added to the other additives described herein, gives 100 percent by weight. The amount in a fully formulated lubricant may be 75 or 80 to 99 percent by weight, or 83 to 98 percent or 85 to 95 percent or 88 to 93 percent. If the oil is present in a concentrate, the amount will be correspondingly less. The amount of diluent oil that typically accompanies the various additives will typically be counted as a part of the oil of lubricating viscosity within the lubricant formulation.

Other additive components may also be present, in conventional amounts as may be typical for the desired application. Particularly noteworthy materials for lubricant applications are described below.

One such component may be a detergent other than the overbased alkylated arylalkyl sulfonates described herein. Detergents in general and their methods of preparation are known and have been described above, in the context of sulfonic acids (sulfonates), carboxylic acids (carboxylates), (hydrocarbyl-substituted) phenols (phenates), phosphonic acids (phosphonates), and mixtures thereof.

Further examples of optional additional detergents include an overbased saligenin detergent. Saligenin detergents are commonly overbased magnesium salts, and they are based on saligenin derivatives. A general example of a saligenin derivative can be represented by the formula 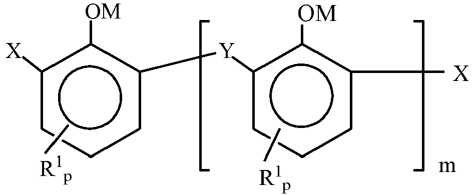 Here, X comprises -CHO or -CH₂OH, Y comprises -CH₂- or -CH₂OCH₂-, and such -CHO groups typically comprise at least 10 mole percent of the X and Y groups. M is hydrogen, ammonium, or a valence of a metal ion such as Mg (that is to say, in the case of a multivalent metal ion, one of the valences is satisfied by the illustrated structure and other valences are satisfied by other species such as anions, or by another instance of the same structure), or mixtures thereof. R¹ is a hydrocarbyl group containing 1 to 60 carbon atoms, m is 0 to typically 10, and each p is independently 0, 1, 2, or 3. At least one aromatic ring contains an R¹ substituent and the total number of carbon atoms in all R¹ groups is at least 7. When m is 1 or greater, one of the X groups can be hydrogen. Saligenin detergents are disclosed in greater detail in U.S. Patent 6,310,009, with special reference to their methods of synthesis (Column 8 and Example 1).

Salixarate detergents are other examples of overbased materials. They may be represented by a linear compound comprising at least one unit of formula (I) or formula (II): 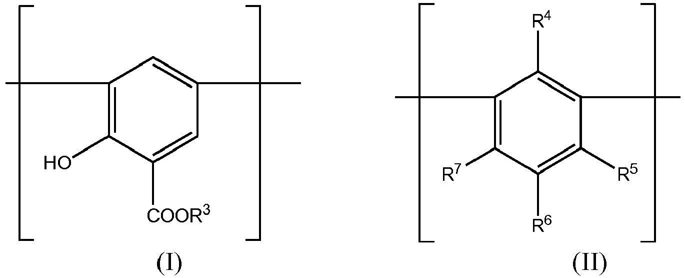 each end of the compound having a terminal group of formula (III) or (IV): 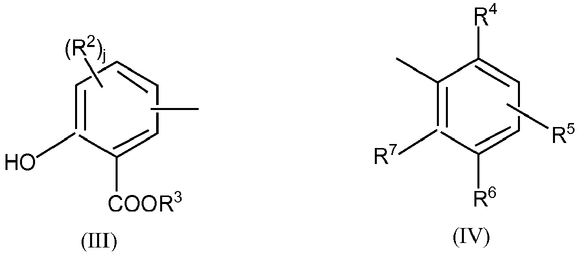 such groups being linked by divalent bridging groups A, which may be the same or different for each linkage. Here, R³ is hydrogen or a hydrocarbyl group or a valence of a metal ion; R² is hydroxyl or a hydrocarbyl group and j is 0, 1, or 2; R⁶ is hydrogen, a hydrocarbyl group, or a hetero-substituted hydrocarbyl group; either R⁴ is hydroxyl and R⁵ and R⁷ are independently either hydrogen, a hydrocarbyl group, or hetero-substituted hydrocarbyl group, or else R⁵ and R⁷ are both hydroxyl and R⁴ is hydrogen, a hydrocarbyl group, or a hetero-substituted hydrocarbyl group. At least one of R⁴, R⁵, R⁶ and R⁷ is hydrocarbyl containing at least 8 carbon atoms. The molecules on average will contain at least one of unit (I) or (III) and at least one of unit (II) or (IV). The ratio of the total number of units (I) and (III) to the total number of units of (II) and (IV) in the composition is 0.1:1 to 2:1. The divalent bridging group "A," which may be the same or different in each occurrence, includes -CH₂- and -CH₂OCH₂-, either of which may be derived from formaldehyde or a formaldehyde equivalent. Salixarate derivatives and methods of their preparation are described in greater detail in U.S. patent number 6,200,936 and PCT Publication WO 01/56968. It is believed that the salixarate derivatives have a predominantly linear, rather than macrocyclic, structure, although both structures are intended to be encompassed by the term "salixarate."

The amount of any additional detergent, if present may be 0.01 to 4 or 0.1 to 3 or 0.5 to 2 weight percent in a fully formulated lubricant. In one embodiment, there is no or substantially no additional detergent present, beside the overbased alkylated arylalkyl sulfonate described in detail above. In certain embodiments the overbased alkylated arylalkyl sulfonate may comprise 75% or more, or 90% or more, or 95% or more, or 98% or more, by weight, of the total detergents, and in other embodiments it may comprise 75% or more, or 90% or more, or 95% or more, or 98% or more by weight of the sulfonate detergents in a composition. In yet other embodiments, the overbased alkylated arylalkyl sulfonate may be present in a smaller relative amount, such as 1 to 90% or 10 to 80% or 20 to 70% or 30 to 60% by weight of the total detergents, the remainder being provided by one or more optional additional detergents.

Dispersants are another type of additive. They are well known in the field of lubricants and include primarily what is known as ashless dispersants and polymeric dispersants. Ashless dispersants are so-called because, as supplied, they do not contain metal and thus do not normally contribute to sulfated ash when added to a lubricant. However they may, of course, interact with ambient metals once they are added to a lubricant which includes metal-containing species. Ashless dispersants are characterized by a polar group attached to a relatively high molecular weight hydrocarbon chain. Typical ashless dispersants include N-substituted long chain alkenyl succinimides, having a variety of chemical structures including typically 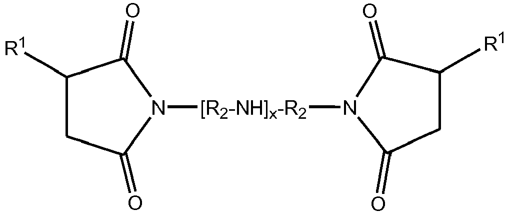 where each R¹ is independently an alkyl group, frequently a polyisobutylene group with a molecular weight (Mn) of 500-5000 based on the polyisobutylene precursor, and R² are alkylene groups, commonly ethylene (C₂H₄) groups. Such molecules are commonly derived from reaction of an alkenyl acylating agent with a polyamine, and a wide variety of linkages between the two moieties is possible beside the simple imide structure shown above, including a variety of amides and quaternary ammonium salts. Also, a variety of modes of linkage of the R¹ groups onto the imide structure are possible, including various cyclic linkages. The ratio of the carbonyl groups of the acylating agent to the nitrogen atoms of the amine may be 1:0.5 to 1:3, and in other instances 1:1 to 1:2.75 or 1:1.5 to 1:2.5. Succinimide dispersants are more fully described in U.S. Patents 4,234,435 and 3,172,892 and in EP 0355895.

It may also be desirable for some formulations that an amount of a hydrocarbyl-substituted succinic anhydride such as polyisobutene succinic anhydride (PIBSA) may be included, that is, the material from which a succinimide dispersant is prepared, prior to reaction with an amine.

Another class of ashless dispersant is high molecular weight esters. These materials are similar to the above-described succinimides except that they may be seen as having been prepared by reaction of a hydrocarbyl acylating agent and a polyhydric aliphatic alcohol such as glycerol, pentaerythritol, or sorbitol. Such materials are described in more detail in U.S. Patent 3,381,022.

Another class of ashless dispersant is Mannich bases. These are materials which are formed by the condensation of a higher molecular weight, alkyl substituted phenol, an alkylene polyamine, and an aldehyde such as formaldehyde. Such materials may have the general structure 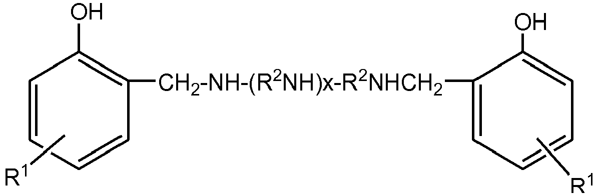 (including a variety of isomers) and are described in more detail in U.S. Patent 3,634,515.

Other dispersants include polymeric dispersant additives, which are generally hydrocarbon-based polymers which contain polar functionality to impart dispersancy characteristics to the polymer.

Dispersants can also be post-treated by reaction with any of a variety of agents. Among these are urea, thiourea, dimercaptothiadiazoles, carbon disulfide, aldehydes, ketones, carboxylic acids, hydrocarbon-substituted succinic anhydrides, nitriles, epoxides, boron compounds, and phosphorus compounds. References detailing such treatment are listed in U.S. Patent 4,654,403.

The amount of any dispersant, if present may be 0.01 to 6 or 0.1 to 5 or 0.5 to 4 or 1 to 3 weight percent in a fully formulated lubricant.

The lubricant may also contain a metal salt of a phosphorus acid. Metal salts of the formula

[(R⁸O)(R⁹O)P(=S)-S]ₙ-M

where R⁸ and R⁹ are independently hydrocarbyl groups containing 3 to 30 carbon atoms, are readily obtainable by heating phosphorus pentasulfide (P₂S₅) and an alcohol or phenol to form an O,O-dihydrocarbyl phosphorodithioic acid. The alcohol which reacts to provide the R⁸ and R⁹ groups may be a mixture of alcohols, for instance, a mixture of isopropanol and 4-methyl-2-pentanol, and in some embodiments a mixture of a secondary alcohol and a primary alcohol, such as isopropanol and 2-ethylhexanol. The resulting acid may be reacted with a basic metal compound to form the salt. The metal M, having a valence n, generally is aluminum, lead, tin, manganese, cobalt, nickel, zinc, or copper, and in many cases, zinc, to form zinc dialkyldithiophosphates. Such materials are well known and readily available to those skilled in the art of lubricant formulation. Suitable variations to provide good phosphorus retention in an engine are disclosed, for instance, in US published application 2008-0015129, see, e.g., claims.

The amount of a metal salt of a phosphorus acid, if present, may be 0.01 to 4 or 0.1 to 3 or 0.5 to 2 weight percent in a fully formulated lubricant.

Another component frequently used is a viscosity modifier. Viscosity modifiers (VM) and dispersant viscosity modifiers (DVM) are well known. Examples of VMs and DVMs may include polymethacrylates, polyacrylates, polyolefins, styrene-maleic ester copolymers, and similar polymeric substances including homopolymers, copolymers and graft copolymers. The DVM may comprise a nitrogen-containing methacrylate polymer and comprise units from a nitrogen-containing methacrylate monomer, for example, a nitrogen-containing methacrylate derived from methyl methacrylate and dimethylaminopropyl amine, i.e., dimethylaminopropyl methacrylamide.

Examples of commercially available VMs, DVMs and their chemical types may include the following: polyisobutylenes (such as Indopol™ from BP Amoco or Parapol™ from ExxonMobil); olefin copolymers (such as Lubrizol™ 7060, 7065, and 7067 from Lubrizol and Lucant™ HC-2000L and HC-600 from Mitsui); hydrogenated styrene-diene copolymers (such as Shellvis™ 40 and 50, from Shell and LZ® 7308, and 7318 from Lubrizol); styrene/maleate copolymers, which are dispersant copolymers (such as LZ® 3702 and 3715 from Lubrizol); polymethacrylates, some of which have dispersant properties (such as those in the Viscoplex™ series from RohMax, the Hitec™ series from Afton, and LZ 7702™, LZ 7727™, LZ 7725™ and LZ 7720C™ from Lubrizol); olefin-graft-polymethacrylate polymers (such as Viscoplex™ 2-500 and 2-600 from RohMax); and hydrogenated polyisoprene star polymers (such as Shellvis™ 200 and 260, from Shell). Viscosity modifiers that may be used are described in U.S. patents 5,157,088, 5,256,752 and 5,395,539. The VMs and/or DVMs may be used in the functional fluid at a concentration of up to 20% by weight. Concentrations of 1 to 12%, or 3 to 10% by weight may be used. The amount of any additional detergent, if present may be 0.01 to 4 or 0.1 to 3 or 0.5 to 2 weight percent in a fully formulated lubricant.

The amount of viscosity modifier and/or dispersant viscosity modifier, if present may be 0.01 to 4 or 0.1 to 3 or 0.5 to 2 weight percent in a fully formulated lubricant.

Another component is an antioxidant. Antioxidants encompass phenolic antioxidants, which may comprise a butyl substituted phenol containing 2 or 3 t-butyl groups. The para position may also be occupied by a hydrocarbyl group or a group bridging two aromatic rings. The latter antioxidants are described in greater detail in U.S. Patent 6,559,105. Antioxidants also include aromatic amine, such as nonylated diphenylamines. Other antioxidants include sulfurized olefins, titanium compounds, and molybdenum compounds. U.S. Pat. No. 4,285,822, for instance, discloses lubricating oil compositions containing a molybdenum and sulfur containing composition. Typical amounts of antioxidants will, of course, depend on the specific antioxidant and its individual effectiveness, but illustrative total amounts can be 0.01 to 5 percent by weight or 0.15 to 4.5 percent or 0.2 to 4 percent. Additionally, more than one antioxidant may be present, and certain combinations of these can be synergistic in their combined overall effect.

Another additive is an antiwear agent. Examples of anti-wear agents include phosphorus-containing antiwear/extreme pressure agents such as metal thiophosphates, phosphoric acid esters and salts thereof, phosphorus-containing carboxylic acids, esters, ethers, and amides; and phosphites. In certain embodiments a phosphorus antiwear agent may be present in an amount to deliver 0.01 to 0.2 or 0.015 to 0.15 or 0.02 to 0.1 or 0.025 to 0.08 percent phosphorus. Often the antiwear agent is a zinc dialkyldithiophosphate (ZDP). For a typical ZDP, which may contain 11 percent P (calculated on an oil free basis), suitable amounts may include 0.09 to 0.82 percent. Non-phosphorus-containing anti-wear agents include borate esters (including borated epoxides), dithiocarbamate compounds, molybdenum-containing compounds, and sulfurized olefins.

Other types of antiwear agents include tartrate esters, tartramides, and tartrimides, such as oleyl tartrimide, as well as esters, amides, and imides of hydroxy-polycarboxylic acids in general. These materials may also impart additional functionality to a lubricant beyond antiwear performance. These materials are described in greater detail in US Publication 2006-0079413 and US Provisional Application 61/120932, filed 9 December 2008.

The amount antiwear agent, if present may be 0.01 to 4 or 0.1 to 3 or 0.5 to 2 weight percent in a fully formulated lubricant.

Other additives that may optionally be used in lubricating oils include pour point depressing agents, extreme pressure agents, color stabilizers and antifoam agents.

Any of a variety of mechanical devices may be lubricated with formulation containing the present overbased materials. Among such devices are engines, including gasoline-fueled, diesel-fueled, alcohol-fueled, bio-diesel-fueled, and hydrogen-fueled engines, as well as hybrid engines and flexible-fueled engines. The engines may be used in passenger cars, in heavy-duty diesel applications, both on-road and off-road, marine diesel application, stationary gas applications, and small engine applications, in both four cycle and two-cycle engines. Other devices include transmissions, such as manual transmissions and automatic transmissions including continuously variable transmissions, traction drive systems, and dual-clutch transmissions. They may also be used in the lubrication of gears, in automotive and industrial applications, in hydraulic systems, as components of greases for various applications, and in metalworking fluids for metal forming or metal cutting.

The amount of each chemical component described is presented exclusive of any solvent or diluent oil, which may be customarily present in the commercial material, that is, on an active chemical basis, unless otherwise indicated. However, unless otherwise indicated, each chemical or composition referred to herein should be interpreted as being a commercial grade material which may contain the isomers, by-products, derivatives, and other such materials which are normally understood to be present in the commercial grade.

As used herein, the term "hydrocarbyl substituent" or "hydrocarbyl group" is used in its ordinary sense, which is well-known to those skilled in the art. Specifically, it refers to a group having a carbon atom directly attached to the remainder of the molecule and having hydrocarbon character. Examples of hydrocarbyl groups include: hydrocarbon substituents, including aliphatic, alicyclic, and aromatic substituents.

It is known that some of the materials described above may interact in the final formulation, so that the components of the final formulation may be different from those that are initially added. For instance, metal ions (of, e.g., a detergent) can migrate to other acidic or anionic sites of other molecules. The products formed thereby, including the products formed upon employing the composition of the present invention in its intended use, may not be susceptible of easy description.

### EXAMPLES

The starting sulfonic acid used in the following examples is a sulfonic acid provided by Champion Technologies, Inc., which is believed to be approximately represented by, or to include species that may be represented by, the general structure

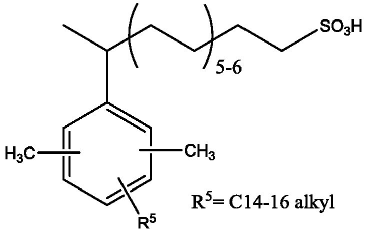

The material has a measured total acid number (TAN, mg KOH/g) of 110.5. It is believed that a small amount of material without the R⁵ alkyl group is also present in the mixture.

### Example 1. Neutral Salt Formation

To a 2L flange flask is charged 600.0 g diluent mineral oil, 69.2 g mixed isobutyl and amyl alcohols, 55.8 g polyisobutenyl succinic anhydride, 60.0 g calcium hydroxide, 17.8 water, and 14.8 g acetic acid. The mixture is heated to 50°C and stirred at 300 r.p.m. under nitrogen. At this time, 500 g of the arylalkyl sulfonic acid is added dropwise at a rate to ensure that the temperature of the mixture does not exceed 50°C. After the addition is complete, the mixture is heated to 100°C for 3 hours and then to 150°C, at which temperature the majority of the volatile solvent is removed by distillation. The product is the neutral calcium salt in diluent oil.

### Example 2. 60 TBN overbased calcium detergent

500.0 g of the neutral salt mixture from Ex. 1, 79.5 g of mixed isobutyl and amyl alcohols, and 63.7 g methanol are heated, with stirring, to 47°C. At this temperature, 20.1 g calcium hydroxide is charged and the mixture is stirred for 20 minutes. Carbon dioxide gas, 5.5 g, is blown through the mixture at this temperature over 30 minutes. Thereafter, the reaction mixture is heated to 150°C to remove volatile solvents and provide a crude mixture with a solids level of 4%. The mixture is filtered through Fax-5™ filter aid to give 451.0g of the desired product (87%), having an analysis of 3.5% calcium, 2.4% sulfur, 59.6 TBN (total base number, as mg KOH/g), 11.7% sulfated ash, and KV100 (kinematic viscosity at 100 °C) of 49.3 mm²/s (cSt).

### Example 3. 290 TBN overbased calcium detergent

500.0 g of the neutral salt mixture from Ex. 1, 26.5 g mixed isobutyl and amyl alcohols, 17.5 g calcium alkylphenate detergent (containing 69% oil), and 52.3 g methanol are heated, with stirring, to 47°C. At this temperature, 34.0 g calcium hydroxide is charged and the mixture is stirred for 20 minutes. Carbon dioxide gas, 1.01 g, is blown through the mixture at this temperature over 30 minutes. A second addition of 34.0 g calcium hydroxide is added and stirred for 20 minutes. An additional amount of CO₂ gas, 20.3 g, is added over 55 minutes. At this point, 150.0 g toluene is added as a cosolvent. A third portion of calcium hydroxide, 34.0 g, is added and the mixture stirred for 20 minutes. Additional CO₂, 20.3 g, is added over 55 minutes. A fourth portion of calcium hydroxide, 34.0 g, is added and the mixture stirred for 20 minutes. Additional CO₂, 15.2 g, is added over 60 minutes. The reaction mixture is heated to 150 °C to remove the volatile solvents to provide a crude mixture with solids content 4.2%. The mixture is filtered through Fax-5™ filter aid to give 397.0g of the desired product (76%), having an analysis of 11.7% calcium, 2.0% sulfur, 290.2 TBN, 39.1% sulfated ash, and KV100 (kinematic viscosity at 100 °C) of 309.2 mm²/s (cSt).

A conventional engine lubricant formulation (except lacking detergent) is prepared, containing, in mineral oil, the indicated amounts in weight percent of the following components (each reported including any diluent oil conventionally present):

| | |
|---|---|
| Viscosity modifier | 6 |
| Dispersant viscosity modifier | 2 |
| Pour point depressant | 0.2 |
| Succinimide dispersant | 8.2 |
| Zinc dialkyl dithiophosphate | 1.09 |
| Sulfur-containing antioxidant | 0.38 |
| Hindered phenolic ester antioxidant | 0.70 |
| Aromatic amine antioxidant | 0.15 |
| Polybutene-succinic anhydride | 0.10 |
| Thiadiazole corrosion inhibitor | 0.02 |
| Antifoam agent | 0.01 |

### Example 4 (reference)

Within the above lubricant formulation is included 1.95 percent of a conventional calcium overbased alkylbenzenesulfonate detergent, 300 TBN, containing 42% diluent oil, 12.0% Ca, 40.8% sulfated ash.

### Example 5

Within the above lubricant formulation is included 1.95 percent of an overbased calcium detergent from an arylalkylsulfonic acid salt, prepared similarly to that of Example 3, 300 TBN, containing 41% diluent oil, 12% Ca, 40.8% sulfated ash.

The lubricant of reference Example 4 and of Example 5 are subjected to the HFRR Ramp Test. This test method comprises a reciprocating steel ball (6 mm) on flat steel disk geometry, with a 1000 µm stroke, 20 Hz frequency and a temperature initially maintained at 40 °C for 15 minutes, then increased to 160 °C at 2 °C per minute. The applied load is 200g. Viscosity properties of the blends and their performance in the HFRR test are summarized in the following table.

| Parameter | Ex 4 (ref) conventional | Ex 5 (present technology) |
|---|---|---|
| Viscosity at 40 °C (mm²/s) | 114.3 | 120.2 |
| Viscosity at 100 °C (mm²/s) | 15.2 | 16.1 |
| Viscosity Index | 139 | 143 |
| Viscosity at -20 °C (cP) | 6007 | 5831 |
| HFRR Wear scar, µm (end of test) | 211 | 200 |
| HFRR Average % film thickness, 40-160°C | 69 | 87 |
| HFRR Avg. coeff. of friction, 40-160°C | 0.140 | 0.137 |
| HFRR Coefficient of friction, 40°C | 0.116 | 0.102 |

The materials of the present technology impart better viscosity performance to the test lubricant than does the comparable reference detergent. They also provide a reduced coefficient of friction and improved wear performance. These performance advantages will contribute to improved fuel economy and engine durability in an engine lubricating using the detergent of the present technology.

The lubricants of reference Example 4 and of Example 5 are further subjected to a soot suspension screening test, which simulates the environment present in diesel engine sumps. The sample lubricant is acidified with 1 vol. % of a 17.4 N mixture of sulfuric acid and nitric acid, and to the mixture is added 6 wt % of a modified carbon black and 5 wt % of a diesel fuel distillation residue. This mixture is subjected to tissumization and sonication to disperse the carbon black, then maintained at 90 °C for 1 week while blown at 0.5 mL/min of 0.27% NOₓ in air. Soot handling is evaluated daily by placing a 0.25 µL aliquot of sample onto chromatography paper and permitting the sample to spread. The diameter of ratio of the diameter of the central carbon spot to the total oil spot is presented as a percentage. Higher percentages indicate greater efficiency of soot dispersion. The central carbon spots are also rated microscopically for uniformity of dispersion. Numerical ratings are assigned based on an algorithm, wherein higher numbers indicate more uniform soot (carbon) dispersion.

| Parameter | Ex 4 (ref) conventional | Ex 5 (present technology) |
|---|---|---|
| Average spot ratio, % | 63 | 71 |
| Numerical spot rating, average | 1.6 | 2.5 |

The overbased salts of the disclosed technology may thus be used to provide improved soot handling performance or improved frictional performance to a lubricant

## Claims

1. An overbased salt comprising:
an anionic portion represented by 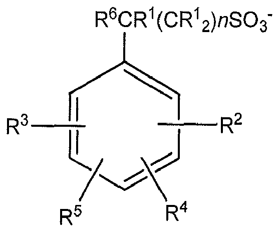 wherein each R¹ is independently H or an alkyl group of 1 to 4 carbon atoms; R², R³, and R⁴ are independently H or hydrocarbyl groups, R⁵ is a group represented by -CR¹R⁷(CR¹₂)*ₒ*CHR¹₂, R⁶ is H or -(CR¹₂)*ₘ*CHR¹₂, and R⁷ is H or -(CR¹₂)*ₚ*CHR¹₂ ; *m*, *n, o*, and *p* are numbers such that *m+n* is 4 to 28 and *o*+*p* is 3 to 27, provided that when R⁶ is *H*, *m* is defined as 0 and *n* is 5 to 29 and when R⁷ is H, *p* is defined as 0 and *o* is 4 to 28; and wherein the total number of carbon atoms in said anionic portion is at least 26; and a metal or amine or ammonium cation;
wherein the overbased salt has a cation content in excess of that which would be present for neutralization according to the stoichiometry of the cation and of said anionic portion.

2. The overbased salt of claim 1 wherein the anionic portion is represented by 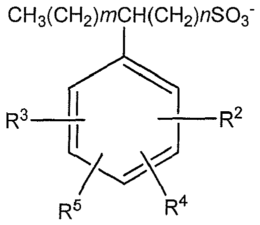 wherein R⁵ is represented by -CHR⁷(CH₂)ₒCH₃ wherein R⁷ is H or -(CH₂)*ₚ*CH₃ and *o*+*p* is 3 to 27.

3. The overbased salt of any of claim 1 or claim 2 wherein the overbased salt is a metal salt having a metal ratio of 1.1 to 40.

4. The overbased salt of any of claims 1 through 3 having a TBN of 10 to 1100 on an oil-free basis.

5. The overbased salt of any of claims 1 through 4 wherein the overbased salt is a carbonated metal salt.

6. The overbased salt of any of claims 1 through 5, wherein the cation comprises a calcium, magnesium, or sodium cation.

7. The overbased salt of any of claims 1 through 6, wherein the R¹ groups in the SO₃⁻ containing substituent are H and *n*+*m* is 8 to 18

8. The overbased salt of any of claim 1 through 7 wherein the R¹ groups in R⁵ are H and *o+p* is 7 to 17.

9. The overbased salt of any of claims 1 through 8 wherein one or two of R², R³, and R⁴ are methyl groups.

10. The overbased salt of any of claims 1 through 9 wherein the anion portion comprises a species represented by 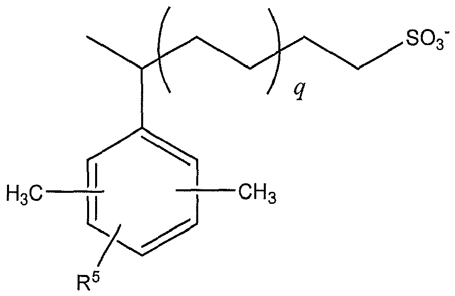 and isomers thereof, wherein q is 5 or 6 and R⁵ is a linear alkyl group containing 14 to 16 carbon atoms.

11. A lubricant composition comprising an oil of lubricating viscosity and the overbased salt of any of claims 1 through 10.

12. The lubricant composition of claim 11 wherein the amount of the overbased salt is 0.01 to 5 percent by weight.

13. The lubricant composition of claims 11 or claim 12 wherein the overbased salt comprises 90 percent or more by weight of the total detergents in the lubricant composition.

14. The lubricant composition of any of claims 11 through 13 further comprising at least one dispersant, friction modifier, antiwear agent, antioxidant, viscosity modifier, or additional overbased salt.

15. A method for lubricating a mechanical device, comprising supplying thereto the lubricant of any of claims 11 through 14.

16. The method of claim 15 wherein the mechanical device comprises an internal combustion engine, a transmission, a gear, or a hydraulic system.

17. A method of preparing an overbased salt, comprising combining a sulfonic acid represented by 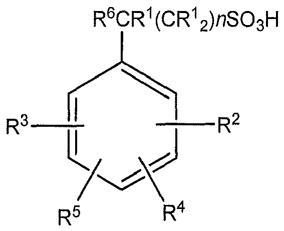 where *n*, *m*, *o*, *p*, R¹, R², R³, R⁴, R⁵, and R⁶ are as defined in any of the previous claims with a stoichiometric excess of a basic metal compound or amine compound or ammonia and optionally reacting said mixture with carbon dioxide.

18. The method of claim 17 wherein the sulfonic acid or reactive equivalent thereof is reacted with an excess of calcium hydroxide, calcium oxide, magnesium hydroxide, magnesium oxide, sodium hydroxide, or sodium oxide.

19. The use of the overbased salt of any of claims 1 through 10 to provide improved soot handling performance or improved frictional performance to a lubricant.

## Patentansprüche

1. Überalkalisiertes Salz, umfassend:
einen anionischen Teil, der durch 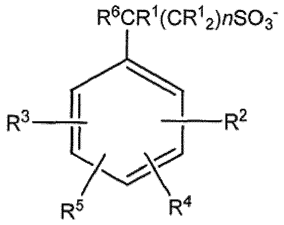 wiedergegeben wird, wobei R¹ jeweils unabhängig für H oder eine Alkylgruppe mit 1 bis 4 Kohlenstoffatomen steht; R², R³ und R⁴ unabhängig für H oder Hydrocarbylgruppen stehen, R⁵ für eine durch -CR¹R⁷(CR¹₂)*ₒ*CHR¹₂ wiedergegebene Gruppe steht, R⁶ für H oder - (CR¹₂)*ₘ*CHR¹₂ steht und R⁷ für H oder -(CR¹₂)*ₚ*CHR¹₂ steht; *m*, *n*, *o* und *p* für solche Zahlen stehen, dass *m* + *n* gleich 4 bis 28 ist und *o* + *p* gleich 3 bis 27 ist, mit der Maßgabe, dass dann, wenn R⁶ für H steht, *m* als 0 definiert ist und n für 5 bis 29 steht, und dann, wenn R⁷ für H steht, *p* als 0 definiert ist und *o* für 4 bis 28 steht; und wobei die Gesamtzahl von Kohlenstoffatomen in dem anionischen Teil mindestens 26 beträgt; und ein Metall- oder Amin- oder Ammoniumkation;
wobei das überalkalisierte Salz einen Kationengehalt aufweist, der über demjenigen liegt, der zur Neutralisation gemäß der Stöchiometrie des Kations und des anionischen Teils vorliegen würde.

2. Überalkalisiertes Salz nach Anspruch 1, wobei der anionische Teil durch 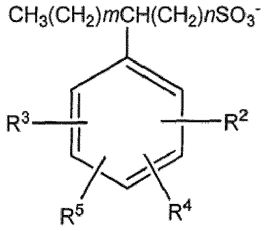 wiedergegeben wird, wobei R⁵ durch -CHR⁷(CH₂)*ₒ*CH₃ wiedergegeben wird, wobei R⁷ für H oder -(CH₂)*ₚ*CH₃ steht und *o* + *p* gleich 3 bis 27 ist.

3. Überalkalisiertes Salz nach Anspruch 1 oder Anspruch 2, wobei es sich bei dem überalkalisierten Salz um ein Metallsalz mit einem Metallverhältnis von 1,1 bis 40 handelt.

4. Überalkalisiertes Salz nach einem der Ansprüche 1 bis 3 mit einer TBN von 10 bis 1100 auf ölfreier Basis.

5. Überalkalisiertes Salz nach einem der Ansprüche 1 bis 4, wobei es sich bei dem überalkalisierten Salz um ein carbonatisiertes Metallsalz handelt.

6. Überalkalisiertes Salz nach einem der Ansprüche 1 bis 5, wobei das Kation ein Calcium-, Magnesium- oder Natriumkation umfasst.

7. Überalkalisiertes Salz nach einem der Ansprüche 1 bis 6, wobei die R¹-Gruppen in dem SO₃⁻ enthaltenden Substituenten für H stehen und *n* + *m* gleich 8 bis 18 ist.

8. Überalkalisiertes Salz nach einem der Ansprüche 1 bis 7, wobei die R¹-Gruppen in R⁵ für H stehen und *o* + *p* gleich 7 bis 17 ist.

9. Überalkalisiertes Salz nach einem der Ansprüche 1 bis 8, wobei eine oder zwei der Variablen R², R³ und R⁴ für Methylgruppen stehen.

10. Überalkalisiertes Salz nach einem der Ansprüche 1 bis 9, wobei der Anionteil eine Spezies, die durch 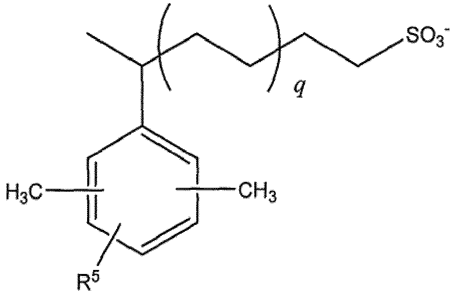 wiedergegeben wird, und Isomere davon umfasst, wobei *q* für 5 oder 6 steht und R⁵ für eine lineare Alkylgruppe mit 14 bis 16 Kohlenstoffatomen steht.

11. Schmiermittelzusammensetzung, umfassend ein Öl mit Schmierviskosität und das überalkalisierte Salz nach einem der Ansprüche 1 bis 10.

12. Schmiermittelzusammensetzung nach Anspruch 11, wobei die Menge des überalkalisierten Salzes 0,01 bis 5 Gewichtsprozent beträgt.

13. Schmiermittelzusammensetzung nach Anspruch 11 oder Anspruch 12, wobei das überalkalisierte Salz 90 Gewichtsprozent oder mehr der gesamten Detergentien in der Schmiermittelzusammensetzung ausmacht.

14. Schmiermittelzusammensetzung nach einem der Ansprüche 11 bis 13, ferner umfassend mindestens ein Dispergiermittel, einen Reibungsmodifikator, ein Verschleißschutzmittel, ein Antioxidans, einen Viskositätsmodifikator oder ein zusätzliches überalkalisiertes Salz.

15. Verfahren zum Schmieren einer mechanischen Vorrichtung, bei dem man dieser das Schmiermittel nach einem der Ansprüche 11 bis 14 zuführt.

16. Verfahren nach Anspruch 15, bei dem die mechanische Vorrichtung einen Verbrennungsmotor, ein Getriebe, ein Zahnrad oder ein Hydrauliksystem umfasst.

17. Verfahren zur Herstellung eines überalkalisierten Salzes, bei dem man eine Sulfonsäure, die durch 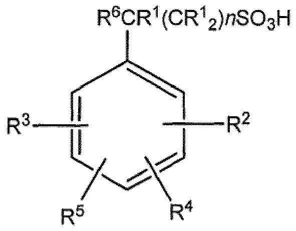 wiedergegeben wird, wobei *n*, *m*, *o*, *p*, R¹, R², R³, R⁴, R⁵ und R⁶ wie in einem der vorhergehenden Ansprüche definiert sind, mit einem stöchiometrischen Überschuss einer basischen Metallverbindung oder Aminverbindung oder Ammoniak vereinigt und die Mischung gegebenenfalls mit Kohlendioxid umsetzt.

18. Verfahren nach Anspruch 17, bei dem man die Sulfonsäure oder das reaktive Äquivalent davon mit einem Überschuss von Calciumhydroxid, Calciumoxid, Magnesiumhydroxid, Magnesiumoxid, Natriumhydroxid oder Natriumoxid umsetzt.

19. Verwendung des überalkalisierten Salzes nach einem der Ansprüche 1 bis 10 zur Ausrüstung eines Schmiermittels mit verbesserter Rußhandhabungsleistung oder verbesserter Reibungsleistung.

## Revendications

1. Sel surbasique comprenant :
une partie anionique représentée par 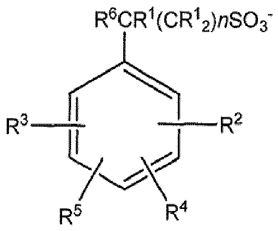 dans laquelle
chaque R¹ est indépendamment H ou un groupe alkyle de 1 à 4 atomes de carbone ;
R², R³ et R⁴ sont indépendamment H ou des groupes hydrocarbyle,
R⁵ est un groupe représenté par -CR¹R⁷(CR¹₂)*ₒ*CHR¹₂,
R⁶ est H ou -(CR¹₂)*ₘ*CHR¹₂ et
R⁷ est H ou -(CR¹₂)*ₚ*CHR¹₂ ;
*m, n, o* et *p* sont des nombres tels que *m*+*n* vaut 4 à 28 et *o*+*p* vaut 3 à 27,
à condition que lorsque R⁶ est H, *m* vaille 0 et *n* vaille 5 à 29 et lorsque R⁷ est H, *p* vaille 0 et *o* vaille 4 à 28 ;
et le nombre total d'atomes de carbone dans ladite partie anionique étant d'au moins 26 ; et
un cation métallique ou d'amine ou ammonium ;
ledit sel surbasique ayant une teneur en cation dépassant celle qui serait présente pour la neutralisation selon la stoechiométrie du cation et de ladite partie anionique.

2. Sel surbasique selon la revendication 1 dans lequel la partie anionique est représentée par 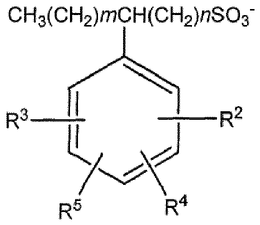 dans laquelle R⁵ est représenté par -CHR⁷(CH₂)*ₒ*CH₃ où R⁷ est H ou -(CH₂)*ₚ*CH₃ et *o*+*p* vaut 3 à 27.

3. Sel surbasique selon l'une quelconque de la revendication 1 ou de la revendication 2, le sel surbasique étant un sel métallique ayant un taux du métal de 1,1 à 40.

4. Sel surbasique selon l'une quelconque des revendications 1 à 3 ayant un indice de base TBN de 10 à 1100 sur une base exempte d'huile.

5. Sel surbasique selon l'une quelconque des revendications 1 à 4, le sel surbasique étant un sel métallique carbonaté.

6. Sel surbasique selon l'une quelconque des revendications 1 à 5, dans lequel le cation comprend un cation calcium, magnésium ou sodium.

7. Sel surbasique selon l'une quelconque des revendications 1 à 6, dans lequel les groupes R¹ dans le substituant contenant SO₃⁻ sont H et *n*+*m* vaut 8 à 18.

8. Sel surbasique selon l'une quelconque des revendications 1 à 7 dans lequel les groupes R¹ dans R⁵ sont H et *o*+*p* vaut 7 à 17.

9. Sel surbasique selon l'une quelconque des revendications 1 à 8 dans lequel un ou deux de R², R³ et R⁴ sont des groupes méthyle.

10. Sel surbasique selon l'une quelconque des revendications 1 à 9 dans lequel la partie anion comprend une espèce représentée par 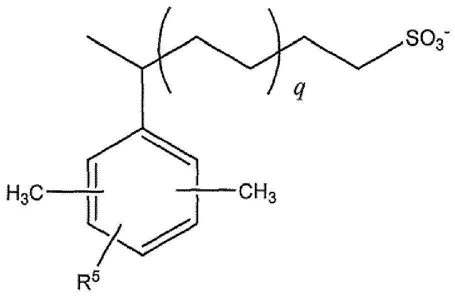 et les isomères de celle-ci, dans laquelle *q* vaut 5 ou 6 et R⁵ est un groupe alkyle linéaire contenant 14 à 16 atomes de carbone.

11. Composition de lubrifiant comprenant une huile de viscosité lubrifiante et le sel surbasique selon l'une quelconque des revendications 1 à 10.

12. Composition de lubrifiant selon la revendication 11 dans laquelle la quantité du sel surbasique est de 0,01 à 5 pour cent en poids.

13. Composition de lubrifiant selon la revendication 11 ou la revendication 12 dans laquelle le sel surbasique constitue 90 pour cent en poids ou plus des détergents totaux dans la composition de lubrifiant.

14. Composition de lubrifiant selon l'une quelconque des revendications 11 à 13 comprenant en outre au moins un dispersant, modificateur de coefficient de frottement, agent antiusure, antioxydant, modificateur de viscosité ou sel surbasique supplémentaire.

15. Procédé pour la lubrification d'un dispositif mécanique, comprenant l'apport à celui-ci du lubrifiant selon l'une quelconque des revendications 11 à 14.

16. Procédé selon la revendication 15 dans lequel le dispositif mécanique comprend un moteur à combustion interne, une transmission, un engrenage ou un système hydraulique.

17. Procédé de préparation d'un sel surbasique, comprenant la combinaison d'un acide sulfonique représenté par 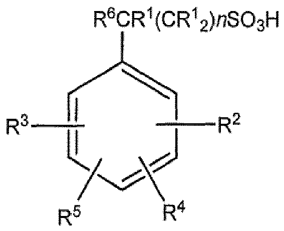 où *n*, *m*, *o*, *p*, R¹, R², R³, R⁴, R⁵ et R⁶ sont tels que définis dans l'une quelconque des revendications précédentes avec un excès stoechiométrique d'un composé métallique basique ou d'un composé amine ou d'ammoniac et éventuellement la réaction dudit mélange avec du dioxyde de carbone.

18. Procédé selon la revendication 17 dans lequel l'acide sulfonique ou un équivalent réactif de celui-ci est amené à réagir avec un excès d'hydroxyde de calcium, d'oxyde de calcium, d'hydroxyde de magnésium, d'oxyde de magnésium, d'hydroxyde de sodium ou d'oxyde de sodium.

19. Utilisation du sel surbasique selon l'une quelconque des revendications 1 à 10 pour conférer une performance de gestion des suies améliorée ou une performance au frottement améliorée à un lubrifiant.
